# EUROPÄISCHE PATENTANMELDUNG

(11) **EP 1 327 438 A1**
(43) Veröffentlichungstag der Anmeldung: **16.07.2003**
(21) Anmeldenummer: 03000177.0
(22) Anmeldetag: 07.01.2003
(51) Int. Cl.: A61K 7/48, A61K 7/40

(54) **kosmetische Zubereitung und ihre Verwendung**

(30) Priorität: 12.01.2002 DE 10200941
(71) Anmelder: Dr. Scheller Cosmetics AG, 73054 Eislingen (DE)
(72) Erfinder: Scheller, Hans-Ulrich, Dr., 73054 Eislingen (DE); Scheller, Alexander, 73054 Eislingen (DE); Sonnenberg, Danuta, 73035 Göppingen (DE); Berger, Marion, 73102 Birenbach (DE)
(74) Vertreter: Werner, Hans-Karsten, Dr.Dipl.-Chem.

(57) **Zusammenfassung**

Eine kosmetische Zubereitung zur präventiven und regenerierenden Behandlung von Skinaging enthält jeweils 0,01 bis 5 Gew.-% Ursolsäure und Oleansäure und/oder Flavonoide.

## Beschreibung

Gegenstand der vorliegenden Erfindung ist eine kosmetische Zubereitung und ihre Verwendung zur präventiven und regenerierenden Behandlung von Skinaging.

Unsere Haut ist unser größtes Organ (ca. 2 Quadratmeter), sie schützt das Körperinnere vor physikalischen, mechanischen und thermischen Einflüssen. Sie verhindert den Verlust von Körperflüssigkeiten und das Eindringen von Mikroorganismen. Die Haut spielt eine wichtige Rolle für die Immunabwehr und dient als wirksamer Filter für das ultraviolette Licht. Ferner verfügt sie über Regulationssysteme für die Temperatur, den Elektrolyt- und Wasserhaushalt. Unter dem Einfluss des Sonnenlichts wird in der Haut das Pigment Melanin und Vitamin D gebildet. Die dünne Körperhülle ist ein komplexes System von verschiedenartigen hochspezialisierten Zellen und Nervenbahnen (Vater Pacini Lamellenkörperchen, Merkel-Zell-Neuritenkomplexe und freien Nervenendigungen), Blutgefäßen und Gerüststoffen, Drüsen und Sensoren. Die sichtbare Oberfläche als oberste Deckschicht von bereits abgestorbenen Hornzellen ist für die Abschirmung gegenüber der Außenwelt verantwortlich und erfüllt somit eine gewisse Schutzfunktion. Von außen nach innen lassen sich 3 Schichten unterscheiden, wobei die Hornschicht, physiologisch betrachtet nur der oberste Teil der relativ dünnen Oberhaut ist, die sich aus weiteren deutlich unterscheidbaren Zellschichten zusammensetzt. Die Lederhaut besteht aus der oberen Papillarschicht und der darunterliegenden Retikularschicht. Ein gut abgestimmtes Geflecht aus Bindegewebe und elastischen Fasern ist in dieser Schicht integriert. Kollagen sorgt v.a. für Festigkeit während Elastin für Elastizität verantwortlich ist.

Selbst wenn es hautfremden Stoffen gelingen sollte die mechanischen und chemischen Barrieren zu überwinden, kommt das eigene Abwehrsystem zum Einsatz. Unser Immunsystem hat mit den Langerhansschen Zellen einen Vertreter in der äußersten Körperhülle. Genauer gesagt stellt die Haut selbst ein immunkompetentes Organ dar. Viele Medikamente und toxisch wirkende Stoffe (u.a. Alkohol und Nikotin) können Unverträglichkeiten bis hin zu Entzündungen, die sich in Rötung, Spannen, Hautbrennen und Juckreiz zeigen, auslösen. Umwelteinflüsse, Stress, falsche Ernährung können wie Chemikalien oder Strahlung die Haut belasten und sogar schädigen. Der natürliche Altersprozess wird dadurch beschleunigt. Man spricht von Skinaging. Dieses umweltbedingte, vorzeitige Altern der Haut (extrinsic aging) kann im Gegensatz zum genetisch festgelegtem Altern (intrinsic aging) positiv beeinflusst und damit verzögert werden. Veränderungen der Haut zeigen sich z.B. in einer Verdickung der Hornhaut, einer verminderten Kollagenbildung, in einer Verdikkung des elastischen Materials (Elastose), in einer verminderten Elastizität, in fahlem Aussehen, in Form von Pigmentflecken (Störung der Pigmentbildung), in faltiger Haut und in einer verzögerten Wundheilung.

Dass der UV-Anteil der Sonnenstrahlung eine schädigende Wirkung auf die Haut ausübt, ist allgemein bekannt. Strahlen im Bereich 290 bis 320 nm (UV-B) können ein Erythem und einen mehr oder weniger starken Sonnenbrand verursachen. Die Wirkung der UV-A-Strahlung wurde in diesem Zusammenhang lange Zeit vernachlässigt (320 bis 400nm). Es wurde angenommen, dass nur die UV-B-Strahlung für die meisten Schäden an der Haut verantwortlich ist, was allerdings durch Studien mittlerweile widerlegt ist. Man weiß heute, dass im Hinblick auf phototoxische Reaktionen und damit Entzündungen der Haut die UV-A-Strahlung ebenfalls gefährlich sein kann, auch weil sie von jahres- und tageszeitlichen Faktoren relativ unabhängig und damit konstant ist. Ein weiterer Grund liegt darin, dass der überwiegende Teil in die Epidermis eindringt, während bei UV-B-Strahlung ein großer Anteil in der Hornschicht absorbiert wird. Zudem kann es zu Synergien von UV-A- und B-Strahlung hinsichtlich oben genannter Beobachtungen kommen. UV-A- und B-Strahlung sorgen für eine Zerstörung des Gewebes resultierend in Falten, geringerer Immunität gegen Infektionen, Hautalterung und Krebs.

Für eine Schädigung der Haut, z.B. in Form von Kollagen- und Elastinfaserveränderungen und damit Strukturveränderungen in der Haut reichen die unter Alltagsbedingungen aufgenommen Strahlungsdosen völlig aus.

Eine Entstehungsursache oben genannter Schädigungen kann in der Wirkungsweise von Radikalen gesehen werden. So spielen beispielsweise bei photochemischen Reaktionsprodukten Hydroxyradikale eine große Rolle. Diese sehr reaktiven und aggressiven Moleküle führen in der Haut zur Bildung von Peroxiden. Diese zerstören die langkettigen Moleküle des Bindegewebes, was letztendlich in Faltenbildung der Haut resultieren kann.

Zum Schutz gegen intensive Sonnenbestrahlung sind zahlreiche Stoffe entwikkelt worden, sogenannte UVA-, B- und Breitbandfilter, die das UV-Licht in den gewünschten Bereichen absorbieren. Sie finden in der Gruppe der Sonnenschutzmittel Ihren Einsatz.

Die vorliegende Erfindung hat sich die Aufgabe gestellt, kosmetische Zubereitungen zur Verfügung zu stellen, die nicht nur eine Prophylaxe für strahlungsbedingten oxidativen Stress erreichen, sondern auch die Ursachen für intrinsische und extrinsische Ursachen des Skinaging beeinflussen. Insbesondere soll eine Linderung bereits eingetretener Hautschädigung in Form von entzündlichen Prozessen erreicht werden.

Ursolsäure ist als entzündungshemmende Substanz bekannt. Als Wirkmechanismen werden unter anderem eine Hemmung der Enzymsysteme der Lipoxygenase und Cyclooxygenase angenommen. Ursolsäure wurde als Wirkstoff vor allem durch die positive Wirkung bei Lebererkrankungen bekannt. Der Wirkmechanismus der Leberprotektion scheint multifaktoriell zu sein und ist im Detail noch nicht geklärt.

Eine weitere günstige Eigenschaft von Ursolsäure liegt in der Hemmung verschiedener Reaktionen in der Tumor-Initiation und -Promotion. Eine positive Wirkung bei topischer Applikation soll hinsichtlich der Hautkrebsprophylaxe bestehen.

Aus der EP 1 161 879 ist bekannt, dass Gemische aus Ursolsäure und Oleansäure geschmacklich verbessert werden können, wenn man von ihnen apolare und niedermolekulare Begleitstoffe abtrennt und dieses gereinigte Gemisch dann in Mono-, Di- oder Triglyceriden löst.

Aus der US 6,162,459 ist bekannt, dass man antivirale Wirkstoffe wie Acychlovir in transdermalen Zubereitungen verbessern kann, wenn man ihnen Verstärker aus der chinesischen Medizin zumischt. Zu diesen Verstärkern zählt Oleansäure und Ursolsäure neben sehr vielen anderen Pflanzen- und Fruchtextrakten.

Aus der US 5,723,139 ist bekannt, dass die Wirksamkeit von Retinol und Retinoiden durch die Zumischung polycyclischer Triterpencarboxylsäure wie Oleansäure, Ursolsäure und Glycyrrhetinsäure verbessert werden kann. Hauptbestandteil ist Retinsäure, die wirksamer ist als Retinol. Glycyrrhetinsäure besitzt einen synergistischen Effekt mit Retinol bei der Inhibierung der Keratinozytendifferenzierung.

Die US 6,022,558 beschreibt transdermale Zubereitungen von Oxicamen als typische Vertreter nichtsteroider antientzündlicher Wirkstoffe. Es wird beschrieben, dass chinesische Kräuteraktivatoren die Absorption beschleunigen. Zu diesen chinesischen Kräuteraktivatoren zählen neben vielen anderen auch Oleansäure und Ursolsäure.

Die US 6,238,678 beschreibt ein Verfahren zur Verbesserung des Erscheinungsbildes der Haut durch Zusatz von Vitamin B3 und seinen Derivaten.

Bei der Aufzählung von zahllosen möglichen zusätzlichen Komponenten werden auch Lichtschutzkomponenten wie Flavonoide aufgezählt, ohne dass dies jedoch einen Niederschlag in den Beispielen gefunden hat.

Flavonoide wirken generell als antioxidativ (v.a. Flavonoide mit mehreren freien OH-Gruppen), antiallergisch und kapillarabdichtend und evtl. auch antiphlogistisch. Je nach ihrer Struktur ist jedoch die eine oder andere Wirkung besonders ausgeprägt.

Einige Flavonoide hemmen Proteinkinasen, die Elastase und Hyaluronidase, Lipoxygenase und Cyclooxygenase. Die in vitro getestete Hemmung der letzten beiden Enzyme steht eng mit der Neutralisierung von Radikalen in Verbindung.

Eine regenerierende Wirkung bei entzündlichen Erscheinungen wird durch Flavonoide nur zu einem geringen Grad ermöglicht. Zudem führt der alleinige Einsatz von Flavonoiden meistens zu einer gelben Färbung der Produkte. Die geringe Löslichkeit setzt zudem Grenzen, was die Konzentration der eingesetzten Flavonoidverbindung betrifft.

Es wurde jetzt gefunden, dass überraschenderweise ein Gemisch aus Ursolsäure und/oder Oleansäure und Flavonoiden eine unerwartet verbesserte Hautschutzwirkung entfaltet, und zwar sowohl in Hinsicht der Prävention als auch der Regeneration der durch extrinsische/intrinsische noxenmikroverletzten Haut. Besonders bewährt haben sich Gemische von Ursolsäure und Oleansäure im Verhältnis 80:20 bis 90:10. Wegen der schlechten Wasserlöslichkeit von Ursolsäure und Oleansäure werden diese vorzugsweise in Form ihrer Natriumsalze eingesetzt.

Das Gemisch enthält bevorzugt Ursolsäure und eine oder beide der Komponenten Oleansäure und Flavanoiden.

Als Flavonoide wiederum haben sich insbesondere bewährt Mischungen aus Rutin und Naringin, die im Mengenverhältnis 1:10 bis 1:2 zum Einsatz kommen.

Besonders deutliche Ergebnisse werden erzielt, wenn die erfindungsgemäß verwendeten Komponenten in Mengen von 0,05 bis 2 Gew.-% vorliegen.

Die Hautschutzwirkung ist deutlich höher als die der Einzelstoffe einschließlich der bekannten Wirkstoffe, wie Pantothenat und Tocopherol. Diese Wirkungssteigerung ist besonders deutlich zu erkennen bei Mischungen, die als Flavonoide, Rutin und Naringin enthalten.

Die Wirkung kann allerdings unterstützt werden durch natürliches Vitamin E und/oder synthetische Tocopherole und/oder Derivate der Tocopherole und/oder anorganische und organische Zink- und/oder Selenverbindungen.

Im Vergleich zu den Einzelsubstanzen bewirkt die erfindungsgemäße Mischung bessere antiinflammatorische Wirkung (Inhibitor der Proteolyse), schnellere Zellerneuerung (repair-Effekt) durch stimulierende Wirkung auf Synthese der Komponenten der extrazellulären Matrix, Verbesserung der Hautstraffung und Elastizität von beanspruchter Haut und verstärkte antioxidative und kräftigende Wirkung.

Als weitere Komponenten können Antioxidantien, wie Ascorbinsäure, Vitamin A und Magnesiumsalze eingesetzt werden.

Oleansäure (3β-hydroxy-olea-12-en-28-oic acid) und ihr Isomer (3β-hydroxyurs-12-en-28-oic acid), die Ursolsäure sind pentazyklische Triterpensäuren, die in natürlichen Pflanzen weit verbreitet sind und oft als Gemische in verschiedenen Verhältnissen vorkommen. Ihre Derivate sind Aglykone natürlich vorkommender Saponine.

Flavonoide sind Glykoside der Flavone, der Flavanone, der 3-Hydroxyflavonone (Flavanole), der Isoflavone. Als besonders wirkungsvoll hat sich die Kombination der Flavonoide Rutin (Quercetin-3-0-rutinosid) und Naringin (Naringenin-7-neohesperidosid) gezeigt.

Erfindungsmäßige Formulierungen im Sinne der vorliegenden Erfindung können als Emulsion vom Typ Öl in Wasser, Wasser in Öl, als Lösungen, multiple Emulsionen vom Typ W/O/W bzw. O/W/O, in Form von Dispersionen auf wässriger oder öliger Basis oder in Gelform vorliegen.

In den nachfolgenden Beispielen sind typische erfindungsgemäße Zubereitungen näher erläutert:

### Beispiel 1 ( O/W Creme)

| | |
|---|---|
| Natriumstearyllactylat | 3,00 |
| Polyglyceryl-3-Stearat | 2,00 |
| Sojaöl | 5,00 |
| Sheabutter | 4,00 |
| Squalan | 4,00 |
| gehärtete pflanzliche Öle | 5,00 |
| Xanthan Gummi | 1,00 |
| Magnesiumaluminiumsilikat | 0,50 |
| Ursolsäure* | 0,20 |
| Rutin | 0,04 |
| Naringin | 0,2 |
| Glycerin | 7,00 |
| Parfüm, Konservierungsstoffe, Antioxidantien | q.s |
| Wasser | ad 100 |

| | |
|---|---|
| * im Gemisch mit Oleansäure im Verhältnis 85:15 als wässrige ethanolische Lösung der Natriumsalze. | |

### Beispiel 2 ( O/W Lotion )

| | |
|---|---|
| Natriumstearyllactylat | 2,00 |
| Polyglyceryl-3-Stearat | 1,00 |
| Glycerinmonodicocoat | 3,00 |
| Sojaöl | 3,00 |
| Sanddornfruchtfleischöl | 1,00 |
| Xanthan Gummi | 0,50 |
| Galactoarabinan | 0,50 |
| Magnesiumaluminiumsilikat | 0,30 |
| Ursolsäure* | 0,20 |
| Rutin | 0,04 |
| Naringin | 0,2 |
| Glycerin | 5,00 |
| Parfüm, Konservierungsstoffe, Antioxidantien | q.s |
| Wasser | ad 100 |

| | |
|---|---|
| * im Gemisch mit Oleansäure im Verhältnis 85:15 als wässrige ethanolische Lösung der Natriumsalze. | |

### Beispiel 3 ( Hydrogel)

| | |
|---|---|
| Carrageenan | 1,50 |
| Gelan Gummi | 0,30 |
| Natriumhyaluronat | 0,20 |
| Alkohol vergällt | 5,00 |
| Glycerin | 10,00 |
| Ursolsäure* | 0,20 |
| Rutin | 0,04 |
| Naringin | 0,2 |
| Parfüm, Konservierungsstoffe, Antioxidantien | q.s |
| Wasser | ad 100 |

| | |
|---|---|
| * im Gemisch mit Oleansäure im Verhältnis 85:15 als wässrige ethanolische Lösung der Natriumsalze. | |

### Beispiel 4 (W/O Creme)

| | |
|---|---|
| Sorbitanoleat, Polyglycerin-3-Ricinoleat | 5,00 |
| Lanolin | 3,00 |
| Mandelöl | 16,00 |
| Jojobaöl | 3,00 |
| Ceresin | 1,00 |
| Magnesiumstearat | 1,00 |
| Magnesiumsulfat | 0,50 |
| Ursolsäure* | 0,20 |
| Rutin | 0,04 |
| Naringin | 0,2 |
| Glycerin | 5,00 |
| Parfüm, Konservierungsstoffe, Antioxidantien | q.s |
| Wasser | ad. 100 |

| | |
|---|---|
| * im Gemisch mit Oleansäure im Verhältnis 85:15 als wässrige ethanolische Lösung der Natriumsalze. | |

## Patentansprüche

1. Kosmetische Zubereitung zur präventiven und regenerierenden Behandlung von Skinaging enthaltend jeweils 0,01 bis 5 Gew.-% Ursolsäure und Oleansäure und/oder Flavonoide.

2. Kosmetische Zubereitung gemäß Anspruch 1, **dadurch gekennzeichnet, dass** Ursolsäure und Oleansäure vorzugsweise im Verhältnis 80:20 bis 90:10 vorliegen.

3. Kosmetische Zubereitung gemäß Anspruch 1 oder 2, **dadurch gekennzeichnet, dass** Ursolsäure und Oleansäure in Form ihrer Natriumsalze vorliegen.

4. Kosmetische Zubereitung gemäß einem der Ansprüche 1 bis 3, **dadurch gekennzeichnet, dass** die Flavonoide aus einer Mischung aus Rutin und Naringin bestehen.

5. Kosmetische Zubereitung gemäß einem der Ansprüche 1 bis 4, **dadurch gekennzeichnet, dass** die Komponenten jeweils in Mengen von 0,05 bis 2 Gew.-% vorliegen.

6. Kosmetische Zubereitung gemäß einem der Ansprüche 1 bis 5, **dadurch gekennzeichnet, dass** sie zusätzlich natürliches Vitamin E und/oder synthetische Tocopherole und/oder Derivate der Tocopherole und/oder anorganische und organische Zink- und/oder Selenverbindungen enthalten.

7. Verwendung der kosmetischen Zubereitung gemäß einem der Ansprüche 1 bis 6 zur Prävention und Regenerierung von extrinsischem Aging.
